# EUROPEAN PATENT SPECIFICATION

(11) **EP 2 603 606 B1**
(45) Date of publication and mention of the grant of the patent: **04.06.2014**
(21) Application number: 11746521.1
(22) Date of filing: 11.08.2011
(51) Int. Cl.: C12Q 1/68

(54) **METHODS AND KITS FOR OF IDENTIFYING A PREMATURE INFANT AT RISK OF HAVING OR DEVELOPING BRONCHOPULMONARY DYSPLASIA**
VERFAHREN UND KITS ZUR IDENTIFIZIERUNG EINES FRÜHGEBORENEN MIT DEM RISIKO, EINE BRONCHOPULMONALE DYSPLASIE AUFZUWEISEN ODER ZU ENTWICKELN
PROCÉDÉS ET KITS POUR IDENTIFIER UN ENFANT PRÉMATURÉ AYANT UN RISQUE D'AVOIR OU DE DÉVELOPPER LA DYSPLASIE BRONCHOPULMONAIRE

(30) Priority: 12.08.2010 EP 10305886
(43) Date of publication of application: 19.06.2013
(73) Proprietor: INSERM - Institut National de la Santé et de la Recherche Médicale, 75013 Paris (FR)
(72) Inventor: DELACOURT, Christophe, F-94010 Creteil Cedex (FR)
(74) Representative: Hirsch, Denise Marie
(86) International application number: PCT/EP2011/063873
(87) International publication number: WO 2012/020102

(56) References cited:
- WO-A2-2010/029545
- US-A1- 2007 021 360
- HILGENDORFF ANNE ET AL: "Association of polymorphisms in the human surfactant protein-D (SFTPD) gene and postnatal pulmonary adaptation in the preterm infant.", ACTA PAEDIATRICA (OSLO, NORWAY : 1992) JAN 2009 LNKD- PUBMED:18785967, vol. 98, no. 1, January 2009 (2009-01), pages 112-117, XP002603286, ISSN: 1651-2227
- KARJALAINEN MINNA K ET AL: "Haplotype analysis of ABCA3: association with respiratory distress in very premature infants", ANNALS OF MEDICINE, vol. 40, no. 1, 2008, pages 56-65, XP009139448, ISSN: 0785-3890
- HILGENDORFF A ET AL: "Association of polymorphisms in the mannose-binding lectin gene and pulmonary morbidity in preterm infants", GENES AND IMMUNITY, vol. 8, no. 8, December 2007 (2007-12), pages 671-677, XP002603288, ISSN: 1466-4879

## Description

### FIELD OF THE INVENTION

The invention relates to methods and kits of identifying a premature infant at risk of having or developing bronchopulmonary dysplasia.

### BACKGROUND OF THE INVENTION

Bronchopulmonary dysplasia (BPD), defined as a requirement for oxygen supplementation at 36 weeks of postmenstrual age (PMA), is the most common chronic respiratory disease in premature infants, and its treatment places major demands on health services. BPD infants are at risk of respiratory morbidity and also have a 2- to 3-fold higher risk of long-term neurodevelopmental disability. Despite considerable obstetric and neonatal advances in the care of very-low-birth-weight (VLBW) infants, BPD continues to occur among 20% to 40% of survivors and accordingly reliable methods for determining the risk of BPD for premature infants are highly recommandable.

Premature birth interrupts normal alveolar and distal vascular development, triggering alveolar hypoplasia and altered microvascular maturation. BPD appears to result from multiple factors that can injure the immature lung and interrupt its development. Factors influencing the risk of BPD in VLBW neonates need to be identified in order to improve preventive measures. Besides the recognized detrimental effects of environmental factors, VLBW twin concordance studies recently suggested a role of genetic factors but few genetic association studies have attempted to identify candidate BPD susceptibility genes.

For example, Hilgendorff A. et al. (2009), Acta Paediatrica vol. 98, no. 1, pages 112-117, discloses an association between the SNP rs1923537 in the SFTPD gene, and the risk of developing respiratory distress syndrome/bronchopulmonary dysplasia in preterm infants.

Moreover, Karjalainen M. et al. (2008), Annals of Medicine, vol. 40, no. 1, pages 56-65 discloses an association between the SNP rs13332514 in the ABCA3 gene, and the risk of developing respiratory distress syndrome/bronchopulmonary dysplasia in preterm infants.

Finally, Hilgendorff A. et al. (2009), Genes and Immunity, vol. 8, no. 8, pages 671-677 discloses an association between SNPs rs1800450 and rs7096206 in the MBP gene, and the risk of developing respiratory distress syndrome/bronchopulmonary dysplasia in preterm infants.

### SUMMARY OF THE INVENTION

The present invention relates to a method of identifying a premature infant at risk of having or developing bronchopulmonary dysplasia, said method comprising detecting in a sample obtained from said premature infant at least one polymorphism in the SPOCK2 gene and/or its associated promoter, wherein the presence of said polymorphism indicates an increased risk of having or developing bronchopulmonary dysplasia.

### DETAILED DESCRIPTION OF THE INVENTION

To identify genetic variants influencing BPD susceptibility, the inventors conducted a genetic association study of a multicenter population, combining pooled-based genome-wide case-control analysis, fine gene mapping, and functional studies. They indeed prospectively collected genomic DNA from 418 premature neonates (postmenstrual age below 28 weeks), of whom 22% had BPD. Two discovery series were created, using a DNA pooling strategy in Caucasian and black African neonates. SNPs significantly associated with BPD were confirmed in an independent replication population. Lung expression of validated genes was evaluated in neonatal rats, in normal and hyperoxic conditions. SPOCK2 was the only gene selected by two analytical strategies in both discovery series. The most significant SNP (rs1245560; p=1.7x10-7) was verified by individual genotyping. Its significant association with BPD was confirmed in the replication population. The adjusted odds ratio (OR) (95% confidence interval) associated with the rs1245560 C allele was 3.57 (1.19-10.76) in BPD neonates. Fine mapping of SPOCK2 in Caucasians identified 5 SNPs in strong linkage disequilibrium, with adjusted ORs of 2.89 to 3.37. Two SNPs were detected by fine mapping in black Africans. rs1245560 remained associated with BPD in all analyses. Evidence of SPOCK2 involvement in lung development was provided by a strong increase in mRNA levels during the alveolar stage in rats and by enhanced SPOCK2 mRNA expression after newborn rat exposure to hyperoxia. SPOCK2 thus represents a new candidate susceptibility gene for BPD, and appears to be involved in normal and pathological alveolar development.

The present invention relates to a method of identifying a premature infant at risk of having or developing bronchopulmonary dysplasia, said method comprising detecting in a sample obtained from said premature infant at least one polymorphism in the SPOCK2 gene and/or its associated promoter, wherein the presence of said polymorphism indicates an increased risk of having or developing bronchopulmonary dysplasia.

"Risk" in the context of the present invention, relates to the probability that an event will occur over a specific time period, as in the conversion to bronchopulmonary dysplasia, and can mean a premature infant's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a premature infant compared either to the absolute risks of low risk cohorts or an average population risk, which can vary by how clinical risk factors are assessed. Odds ratios, the proportion of positive events to negative events for a given test result, are also commonly used (odds are according to the formula p/(1-p) where p is the probability of event and (1- p) is the probability of no event) to no- conversion. Alternative continuous measures which may be assessed in the context of the present invention include time to bronchopulmonary dysplasia conversion and therapeutic bronchopulmonary dysplasia conversion risk reduction ratios.

"Risk evaluation," or "evaluation of risk" in the context of the present invention encompasses making a prediction of the probability, odds, or likelihood that an event or disease state may occur, the rate of occurrence of the event or conversion from one disease state to another, i.e., from a normal condition to bronchopulmonary dysplasia condition. Risk evaluation can also comprise prediction of future clinical parameters, traditional laboratory risk factor values, or other indices of bronchopulmonary dysplasia, such as duration of ventilation support or requirement for long-term oxygen supply. The methods of the present invention may be used to make continuous or categorical measurements of the risk of conversion to bronchopulmonary dysplasia, thus defining the risk spectrum of a category of premature infants defined as being at risk for bronchopulmonary dysplasia. In the categorical scenario, the invention can be used to discriminate between normal and other premature infant cohorts at higher risk for bronchopulmonary dysplasia. In other embodiments, the present invention may be used so as to discriminate those having early late stages of bronchopulmonary dysplasia or late stages of bronchopulmonary dysplasia from normal.

A "sample" in the context of the present invention is a biological sample isolated from a premature infant and can include, by way of example and not limitation, bodily fluids and/or tissue extracts such as homogenates or solubilized tissue obtained from a premature infant. Tissue extracts are obtained routinely from tissue biopsy and autopsy material. Bodily fluids useful in the present invention include blood, urine, saliva or any other bodily secretion or derivative thereof. As used herein "blood" includes whole blood, plasma, serum, circulating epithelial cells, constituents, or any derivative of blood. In particular the sample may consist of an umbilical cord sample.

In one embodiment of the invention, the premature infant may be healthy at birth, but considered at risk for developing bronchopulmonary dysplasia, based on clinical indicia such as gestational age, intrauterine growth retardation, twin pregnancy, requirement for exogenous surfactant, patent ductus arteriosus, associated sepsis. In another embodiment, said premature infant may also be free of said clinical indicia but develop chronic respiratory symptoms which could be related to bronchopulmonary dysplasia.

As used herein, the term "SPOCK2 gene" has its general meaning in the art and refers to the gene encoding for SPARC/osteonectin, CWCV, and Kazal-like domains proteoglycan 2, also known as Testican-2.

The terms "mutant" and "mutation" mean any detectable change in genetic material, e.g. DNA, RNA, cDNA, or any process, mechanism, or result of such a change. This includes gene mutations, in which the structure (e.g. DNA sequence) of a gene is altered, any gene or DNA arising from any mutation process, and any expression product (e.g. protein or enzyme) expressed by a modified gene or DNA sequence. Generally a mutation is identified in a premature infant by comparing the sequence of a nucleic acid or polypeptide expressed by said premature infant with the corresponding nucleic acid or polypeptide expressed in a control population. A mutation in the genetic material may also be "silent", i.e. the mutation does not result in an alteration of the amino acid sequence of the expression product.

The term "polymorphism" or "allelic variant" means a mutation in the normal sequence of a gene, Allelic variants can be found in the exons, introns, or the coding region of the gene, or in the sequences that control expression of the gene. Complete gene sequencing often identifies numerous allelic variants (sometimes hundreds) for a given gene. The significance of allelic variants is often unclear until further study of the genotype and corresponding phenotype occurs in a sufficiently large population.

In a particular embodiment, the present invention relates to a method of identifying a premature infant at risk of having or developing bronchopulmonary dysplasia, wherein said method comprises a step consisting of detecting in a sample obtained from said premature infant the single nucleotide polymorphism rs1245560 in the SPOCK2 gene, wherein the presence of allele (C) of rs1245560 indicates an increased risk of being at risk of having or developing bronchopulmonary dysplasia.

The term "Allele" has the meaning which is commonly known in the art, that is, an alternative form of a gene (one member of a pair) that is located at a specific position on a specific chromosome which, when translated result in functional or dysfunctional (including non-existant) gene products.

The term "Single nucleotide polymorphism" or "SNP" means a single nucleotide variation in a genetic sequence that occurs at appreciable frequency in the population. There are millions of SNPs in the human genome. Most commonly, these variations are found in the DNA between genes. When SNPs occur within a gene or in a regulatory region near a gene, they may play a more direct role in disease by affecting the gene's function.

All the SNPs pertaining to the invention (e.g. rs1245560, rs1245576, rs1049269, rs748035, rs1245558, or rs1245540) are known per se and sequences of them are publicly available from the data base http://www.ncbi.nlm.nih.gov/SNP/ or http://hapmap.ncbi.nlm.nih. gov/.

SNP rs1245560 (amibguity code **M** = A/C) is identified in the following sequence:

CCAGGCAGGAGTGCAGTGGTGCAATCATAGCTCACTGTAGCCCCGAACTCCCAGGCTCAA
GCGATCCTCCCACCTCAGCCTTCCAAGTACCTGGGAGCACAGGCATGTGCCACCATGCCT
GTCTAATTTTTATTGTGGTGGTTTTTTTCATAGAGATAGGGTCTTGATATGCTGCCCAGG
CTGATCTCGAACTCCTGGGCTCAAGTGATCCTCCCTCAGCTCCCAAAGCACTGGGG
TTACAGGCATGAGCCACTGTGCCCTTCCCCAAGGAGCTTTTTAAACCTTCCAGCACGCAC
ATGCTACCTGAGACCCCTGAAATCCCATGGGTGGTACCCAGCTCGGGCCACGCTTCTGTG
CTGGCTCTAGGCAGGCAGCTTAGAGACCACTGGGCCAGCCATCAGGCCTCAGGTCCCTCC
CGCTCTGTGCTTATGTCTAATATTTTGCTTATGTTCTTGGGTGTCTTCACCCCAAACACG
GGGCCTCTGGGATGAAAAAGMACAGCCATTCTTCCTCCCTCCGAGGGTGCCTCACACAAA
GCGATACAAGGTGTGCTGAGGAGCAGGCCGGCCCTGGGAGAACCCAGGCTCCTGCTTCCA
ATTCCAGGCCCGTTCCTGGAGCACGCTTACCGTGCCACCCTCACCAACCCAAAGACTGTC
CTGCCCCTTCCCCTCCCGAATCCCTCTCGCTTGCCCTCCCT

SNP rs1245576 (amibguity code **Y** = T/C) is identified in the following sequence:

TCACTGCATTCCATCCACCTGGCAACAGTAACGGATCCAATCAGGGTCCTTCCCTGGGAT
TAATATGCAGATTTCGGGTGCAACCAGCTCTCTCTGTCTGCTGGGGCAGTCTTGTTCCCT
GCTACATGGAGACAGCCCATCAGGAGTCAGACAGAATGAGGCCAACAGGTGAAAAGAAAC
AGTAAAGCAAAGACGGTCAGCATGGAGGAGCCCTGGGGTGCTGAACTCACAGTTGCCATC
CCCAAAGACCTGGTTCCTCAAATTCTTCCTTCAATTCCAAGACTTGCTGCAGGATCCCCA
YCTTCTGTGAGAGCCAATACATTATTTCTCTGTCTAAGCCAGTCAGAGTTAGGTAGGTGT
CTGTCACTTGCAACCAAAGGCTCTTGGCTGGTACAGTCCTGTTTCTAAAACCCTGCCCCT
GCTGACCAAGCAACCAGTGAAAGCAAAGGGAGACGGTGCCCATCCTGGCAGCAGCCCTCT
CTCCGGTTCAGACAGGTCACTCCAGGGCACTTTCTGATGGGTGCTTAGTGCTGAAAAGGG
TGACCCTGAAAAAGCACAGGGATGTGAATTGAAGTCAATTATGAATAGGGTAAGAGAAGA
T

SNP rs1049269 (amibguity code **R** = A/G) is identified in the following sequence:

TTCTCTGCTGCAACCTACCAGATCTGACATCCACCTCCCCCAGCACCCATGGCCAAGGA
GGCCTGGGGCAGCCAAGGGGAGTTCCAGGACCAAGCAAGCAAGAAACCGTTCTTTGAACA
CATGGTTAAGCTTCTTCCAGCATGGCCCTAATTCCCCTACCTGCCTAAGCCAGGGGAGTG
GGGCAGGAAGGAGCGAGGCATCGAGGAGAGGGCACTGGAGGGGCTGGGTGGCAGGCCCC
TTGGTCAAGGTCACAAATCAAGGAGCTGAGGAAGGACCGATCTGCAACCTTGCTCATGAG
GAAACCTTAACCTTGTCTAACAAAAAGCCACTTTGAGGCCGCCCTTCAGCTCCACAGGGA
GCTGCTGTTTTGTCTACCTTTTGAGGGGACCAGTCTCAGTRGGGCGAAAGGCAGTGTGGG
GGCTTCGGAAACAGGCAAGGCTGGACCCCTGCAATCCTACCAGAGGCTAGTTCAGTCCCT
TTTGCTTTCTTCCTAAAACATATTTACAAGGACACACAAACGTGCACAGCCAGAAGACCC
AGAGACAGAGAGAGCACGTGTCCCAGGAGCGGAGGGTGACATCAAGAGGACAGGGGACAC
CCTTCCTCTGAACTCCTTCCTTCCCCAGGCCCCCCAGCATCCTTAACAACCCCCACCCCC
ACCCCGAGGAGGAAAGTTCCAGAACTCCAGCTGGGACAAATGGAAGAGATAGGTAGACTC
CAATCCTAGCAAGAGCAGAAGTTTCAAGTAGCTCCGAGTCAGAGGCGCGGCTGCCTCAGA
GAGCAGAGGGCTGATGTGGAA

SNP rs748035 (amibguity code **Y** = C/T) is identified in the following sequence:

TGGGGATATCAGTGTGAGGCCCTCTGGTGTTCTGGGGTCCCTTGCCTCATCATGTGCCGA
GCAATGGCCTTCGATGGCTGTGTTCCTCTGACCCCCAGGAACCCCAATAAGAGGCCAGTA
TTCTTATGTCTTCATCACAGAGCTCAAGACTGGCCTTAGGAGTAGCAGGGGCCCGTGGCT
TTGAGAGGGTGGGGCACCCCCACTCCGTTCCCAGTCCAGCCCCAGCCCCACCACCAGGAC
AGATGTCCGAGGTGCCCATGCTGCGTAGGAACAGAGCTTCTCATGCCTATGCCCTGGGTG
TCTCAGAAACTGCCTGAGAAACGGTGCACTGTCTGTCTCCTGCCAAGGCCCCTTCCTTGT
CAGTCCCCACTCCTGACCTCTGCCCACCATGTGAGTGAGGTGGGAGGAAGACCCCCTCAC
CCAGTCTGCAAAGGGATGCTTTTTGCTCAAGGTCTGACATCTCTGCAGAGCTCAGCTCAT
ACACGGAGTGAGGGAGGAGGCCATGCCTGTGATGAGGCTTGCCCAGTTATCACGATTCAG
CTTCAAACCCTCAGAGGCAGAGGACCCAGAAAGGGTCCTACGAGGTCACCTGGTCCAGCC
CCCCCTTTGTGCCGGAGAAAGCYCAGAGACAGATGACCTGACCCAGGTCCTCCCGTCACA
CAACCCAGGTCTCCCGATGCCCAGCACAGACCACTTTTCTTGCTACAACACACTCTCTGA
GGCCTAAGGCACAAGGGAGACCTTCTCACCCTGCTCCA

SNP rs1245558 (amibguity code **Y** = T/C) is identified in the following sequence:

GAGACCTCACCCCCTACACCCCTCACCACCAAGGACTCAGGGGCCTGGGCTCCTGCTTGG
CCTTGCCACTGACTGCCAGCAAGTGAGTCGCTTCCCTTGCTGGACTGGCTTCCTTGGCTG
TAAAATAAGGATGACCCCCTTGCCTTCCCCTCTGAGCTGCAGAAGAACCCAGGCAATCAT
GTCTGCAAGGGGACTTTGTGAACTGAAGCTCCATACTTTCAAAAGACACAAGACAGGACA
CCTGAACCCCGGGTTCTCTGGCACACACCCGGGGAGCCTAGGGACAGGACTGGGCAGAGG
YTCTGTAGGGTCTCAGTGTTACAAGGGGTCTCAATATGGACATCTGACATTGTCTCCACT
TGAGGACACACAGCCAGAGGCACAGCGACAGGACGGAAAGAAGACAGTCATCATAAGACT
GAATACACCACAATTTCCCTAACCCTGTTACGGCATTTCTTCATTTTTAATAGATCAAG
AATCTCTAAAAATGGAAGTGGCCAGGCCTGGCTCAGCCTCTGAGAGGCCCTGTATCTGC
CATTATTTTGGCCTCATTCCTCAGGCAACGGGGCCTGTGATGGAGGCCAGTCCACCGAAG
A

SNP rs1245540 (amibguity code **Y** = C/T) is identified in the following sequence:

AATGCCTCAGTGGCTCAGGCCGGAGTCTCGTTGGAAAACAGAAAACAATAGCTCCCTTT
CCATGTTTTCTTGTTTCCTTTGCTGTTGGATTTAGTGGTGGTGCTGCAGTGAGGCCCTGG
CCATTGACGTCTTCATCTCACAGTAATAAAGCATTCTTCCTGGGTGTGTCCTGGCGCTGT
ACAGTGAGAGAGCACGGCTTGCTGTCGTGGAGACTAGAGGACAGAATCCCACTTACTGGC
TGGATTCTTGAGCAATTACCTATTTCTATCTAAAACTAATGGTAATACCATCATTAAAA
YTGCTGTAAAGTGCAAGCAAAATTACATACTAAGTGGGCATAATGGGCATTTTTTTAAAA
AGTTAGTCTTTCCTCTGGTTGGGAAGAGAAGGCTACGGTGTCCCATAGTTCTGAAGATTC
ATCAAAATTTGCCATCTTTTCCACCATCTCAATCCTACAATCCTAGTTTTTGTTGTTTT
TTTTTTTTTTGTTTCTGTTTTCGTTTTAGCAAAGCTAGCTATATATCTCTAAGGCACTT
AGGAGATAGGTACCAGATACCACAGCTCTCCCATTCCTGTGCTATCTTAGGGGCTTCCAG
A

In a particular embodiment, the method of the invention may further comprise a step consisting of detecting at least one polymorphism in linkage disequilibrium with rs1245560 wherein said polymorphism is selected from the group consisting of rs1245576, rs1049269, rs748035, rs1245558, rs1245540 and wherein ;
- the presence of the allele C of rs1245576
- the presence of the allele G of rs1049269
- the presence of the allele A of rs748035
- the presence of the allele C of rs1245558
- the presence of the allele G of rs1245540
indicate an increased risk of having or being at risk of having or developing bronchopulmonary dysplasia.

The term "linkage disequilibrium" (LD) refers to a population association among alleles at two or more loci. It is a measure of co-segregation of alleles in a population. Linkage disequilibrium or allelic association is the preferential association of a particular allele or genetic marker with a specific allele, or genetic marker at a nearby chromosomal location more frequently than expected by chance for any particular allele frequency in the population.

According to the invention, the determination of the presence or absence of said polymorphism may be determined by nucleic acid sequencing, PCR analysis or any genotyping method known in the art. Examples of such methods include, but are not limited to, chemical assays such as allele specific hybridation, primer extension, allele specific oligonucleotide ligation, sequencing, enzymatic cleavage, flap endonuclease discrimination; and detection methods such as fluorescence, chemiluminescence, and mass spectrometry.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989).

The conditions of temperature and ionic strength determine the "stringency" of the hybridization. For preliminary screening for homologous nucleic acids, low stringency hybridization conditions, corresponding to a Tm (melting temperature) of 55°C, can be used, e.g., 5x SSC, 0.1 % SDS, 0.25 % milk, and no formamide ; or 30 % formamide, 5x SSC, 0.5 % SDS). Moderate stringency hybridization conditions correspond to a higher Tm, e.g., 40 % formamide, with 5x or 6x SCC. High stringency hybridization conditions correspond to the highest Tm, e.g., 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989 I1.7-11.8). A minimum length for a hybridizable nucleic acid is at least about 10 nucleotides, preferably at least about 15 nucleotides, and more preferably the length is at least about 20 nucleotides.

In a specific embodiment, the term "standard hybridization conditions" refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

As used herein, an amplification primer is an oligonucleotide for amplification of a target sequence by extension of the oligonucleotide after hybridization to the target sequence or by ligation of multiple oligonucleotides which are adjacent when hybridized to the target sequence. At least a portion of the amplification primer hybridizes to the target. This portion is referred to as the target binding sequence and it determines the target-specificity of the primer. In addition to the target binding sequence, certain amplification methods require specialized non-target binding sequences in the amplification primer. These specialized sequences are necessary for the amplification reaction to proceed and typically serve to append the specialized sequence to the target. For example, the amplification primers used in Strand Displacement Amplification (SDA) include a restriction endonuclease recognition site 5' to the target binding sequence (US Patent No. 5,455,166 and US Patent No. 5,270,184). Nucleic Acid Based Amplification (NASBA), self-sustaining sequence replication (3SR) and transcription based amplification primers require an RNA polymerase promoter linked to the target binding sequence of the primer. Linking such specialized sequences to a target binding sequence for use in a selected amplification reaction is routine in the art. In contrast, amplification methods such as PCR which do not require specialized sequences at the ends of the target, generally employ amplification primers consisting of only target binding sequence.

As used herein, the terms "primer" and "probe" refer to the function of the oligonucleotide. A primer is typically extended by polymerase or ligation following hybridization to the target but a probe typically is not. A hybridized oligonucleotide may function as a probe if it is used to capture or detect a target sequence, and the same oligonucleotide may function as a primer when it is employed as a target binding sequence in an amplification primer. It will therefore be appreciated that any of the target binding sequences disclosed herein for amplification, detection or quantisation of SPOCK2 may be used either as hybridization probes or as target binding sequences in primers for detection or amplification, optionally linked to a specialized sequence required by the selected amplification reaction or to facilitate detection.

For example, the presence or absence of said polymorphism may be detected in a DNA sample, preferably after amplification. For example DNA may be amplified, after which a mutated site may be detected in the amplified sequence by hybridization with a suitable probe or by direct sequencing, or any other appropriate method known in the art..

Actually numerous strategies for genotype analysis are available. Briefly, the nucleic acid molecule may be tested for the presence or absence of a restriction site. When a base polymorphism creates or abolishes the recognition site of a restriction enzyme, this allows a simple direct PCR test for the polymorphism. Further strategies include, but are not limited to, direct sequencing, restriction fragment length polymorphism (RFLP) analysis; hybridization with allele-specific oligonucleotides (ASO) that are short synthetic probes which hybridize only to a perfectly matched sequence under suitably stringent hybridization conditions; allele-specific PCR; PCR using mutagenic primers; ligase-PCR, HOT cleavage; denaturing gradient gel electrophoresis (DGGE), temperature denaturing gradient gel electrophoresis (TGGE), single-stranded conformational polymorphism (SSCP) and denaturing high performance liquid chromatography. Direct sequencing may be accomplished by any method, including without limitation chemical sequencing, using the Maxam-Gilbert method ; by enzymatic sequencing, using the Sanger method ; mass spectrometry sequencing ; sequencing using a chip-based technology; and real-time quantitative PCR. Preferably, DNA from a subject is first subjected to amplification by polymerase chain reaction (PCR) using specific amplification primers. However several other methods are available, allowing DNA to be studied independently of PCR, such as the rolling circle amplification (RCA), the InvaderTMassay, or oligonucleotide ligation assay (OLA). OLA may be used for revealing base polymorphisms. According to this method, two oligonucleotides are constructed that hybridize to adjacent sequences in the target nucleic acid, with the join sited at the position of the polymorphism. DNA ligase will covalently join the two oligonucleotides only if they are perfectly hybridized.

Therefore, useful nucleic acid molecules, in particular oligonucleotide probes or primers, according to the present invention include those which specifically hybridize the regions where the polymorphisms are located.

Oligonucleotide probes or primers may contain at least 10, 15, 20 or 30 nucleotides. Their length may be shorter than 400, 300, 200 or 100 nucleotides.

According to a further embodiment said polymorphism in the SPOCK2 gene may be detected at the protein level.

Said polymorphism may be detected according to any appropriate method known in the art. In particular a sample obtained from a subject may be contacted with antibodies specific of the mutated form of SPOCK2 which results from the polymorphism according to the invention, i.e. antibodies that are capable of distinguishing between a mutated form of SPOCK2 and the wild-type protein (or any other protein), to determine the presence or absence of a SPOCK2 specified by the antibody.

Antibodies that specifically recognize a mutated SPOCK2 also make part of the invention. The antibodies are specific of mutated SPOCK2 that is to say they do not cross-react with the wild-type SPOCK2.

The antibodies of the present invention may be monoclonal or polyclonal antibodies, single chain or double chain, chimeric antibodies, humanized antibodies, or portions of an immunoglobulin molecule, including those portions known in the art as antigen binding fragments Fab, Fab', F(ab')2 and F(v). They can also be immunoconjugated, e.g. with a toxin, or labelled antibodies.

Whereas polyclonal antibodies may be used, monoclonal antibodies are preferred for they are more reproducible in the long run.

Procedures for raising "polyclonal antibodies" are also well known. Polyclonal antibodies can be obtained from serum of an animal immunized against the appropriate antigen, which may be produced by genetic engineering for example according to standard methods well-known by one skilled in the art. Typically, such antibodies can be raised by administering mutated SPOCK2 subcutaneously to New Zealand white rabbits which have first been bled to obtain pre-immune serum. The antigens can be injected at a total volume of 100 µl per site at six different sites. Each injected material may contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed by Harlow et al. (1988) which is hereby incorporated in the references.

A "monoclonal antibody" in its various grammatical forms refers to a population of antibody molecules that contains only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art. Monoclonal antibodies (mAbs) may be prepared by immunizing purified mutated SPOCK2 into a mammal, e.g. a mouse, rat, human and the like mammals. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody.

While mAbs can be produced by hybridoma culture the invention is not to be so limited. Also contemplated is the use of mAbs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No.; European Patent Publications No. 0239400 to Winter et al. and No. 0125023 to Cabilly et al.

Antibody generation techniques not involving immunisation are also contemplated such as for example using phage display technology to examine naive libraries (from non-immunised animals).

Antibodies raised against mutated SPOCK2 may be cross reactive with wild-type SPOCK2. Accordingly a selection of antibodies specific for mutated SPOCK2 is required. This may be achieved by depleting the pool of antibodies from those that are reactive with the wild-type SPOCK2, for instance by submitting the raised antibodies to an affinity chromatography against wild-type SPOCK2.

Alternatively, binding agents other than antibodies may be used for the purpose of the invention. These may be for instance aptamers, which are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

Probe, primers, aptamers or antibodies of the invention may be labelled with a detectable molecule or substance, such as a fluorescent molecule, a radioactive molecule or any others labels known in the art. Labels are known in the art that generally provide (either directly or indirectly) a signal.

The term "labelled", with regard to the probe, primers, aptamers or antibodies of the invention, is intended to encompass direct labelling of the the probe, primers, aptamers or antibodies of the invention by coupling (i.e., physically linking) a detectable substance to the the probe, primers, aptamers or antibodies of the invention, as well as indirect labeling of the probe, primers, aptamers or antibodies of the invention by reactivity with another reagent that is directly labeled. Other examples of detectable substances include but are not limited to radioactive agents or a fluorophore (e.g. fluorescein isothiocyanate (FITC) or phycoerythrin (PE) or Indocyanine (Cy5)). Examples of indirect labeling include detection of a primary antibody using a fluorescently labeled secondary antibody and end-labeling of a DNA probe with biotin such that it can be detected with fluorescently labeled streptavidin. An antibody or aptamer of the invention may be labelled with a radioactive molecule by any method known in the art. For example radioactive molecules include but are not limited radioactive atom for scintigraphic studies such as I123, I124, In111, Re186, Re188.

A further object of the invention is a kit for performing the methods of the invention, comprising at least one primer and/or at least one probe for amplification of a sequence comprising the polymorphisms of the invention and instructions for use.

In one embodiment of the invention, the primer or probe may be labelled with a suitable marker. In another embodiment of the invention, the primer or probe may be coated on an array.

Alternatively, the kit of the invention may comprise a labelled compound or agent capable of detecting the mutated polypeptide of the invention (e.g., an antibody or aptamers as described above which binds the polypeptide). For example, the kit may comprise (1) a first antibody (e.g., attached to a solid support) which binds to a polypeptide comprising a mutation of the invention; and, optionally, (2) a second, different antibody which binds to either the polypeptide or the first antibody and is conjugated to a detectable agent.

The kit can also comprise, e.g., a buffering agent, a preservative, or a protein stabilizing agent. The kit can also comprise components necessary for detecting the detectable agent (e.g., an enzyme). Each component of the kit is usually enclosed within an individual container and all of the various containers are within a single package along with instructions for observing whether the tested subject is suffering from or is at risk of developing an bronchopulmonary dysplasia.

The kit can include clinical data such as phenotype of the macula and questionnaire of the premature infant.

The method of the invention may be used to choose the accurate treatment for said premature infant. For example, premature infants identified to be at risk for bronchopulmonary dysplasia may thus receive an accurate treatment based on the administration of corticoids that may help lung maturation. Moreover the methods of the invention may help the physicians to adapt the therapeutic care of the premature infant and to avoid them to administer said infants with drugs that may be deleterious for their development. It has been indeed shown that corticoids to provide many adverse side effects, particularly on the neuronal development. Such methods may thus help the physician to make a choice on a therapeutic treatment. Costs of the treatments may therefore be adapted to risk of the premature infants.

The present invention also provides novel targets and methods for the screening of drug candidates or leads. Such drug candidates or leads are useful for developing a treatment for bronchopulmonary dysplasia. The methods include binding assays and/or functional assays, and may be performed in vitro, in cell systems, in animals, etc. Functional assays comprise, but are not limited to, in vitro assays, cell-based assays or animal-based assays that involve a SPOCK2 protein.

The invention relates to methods for screening of compounds that modulate the SPOCK2 activity or expression. Such compounds, for example, can increase or decrease affinity and/or rate of interfering of the SPOCK2 protein to metalloproteases, compete with metalloproteases for interfering to the SPOCK2 protein, or displace metalloproteases bound to the SPOCK2 protein. Preferably, the invention concerns methods for screening of compounds that increase or restore the natural SPOCK2 activity or expression. By "natural" SPOCK2 activity is intended the activity of the wild-type SPOCK2 protein. Furthermore, the invention concerns methods for screening of compounds that modulate the activity of the altered SPOCK2.

Therefore, the present invention concerns a method of selecting biologically active compounds, said method comprising contacting a test compound with an altered SPOCK2 gene or an altered SPOCK2 protein or fragment thereof of at least 15 consecutive residues comprising an alteration, wherein the alteration reduces, modifies, or abolishes the activity of SPOCK2, and determining the ability of said test compound to modulate the expression and/or activity of said gene or protein or fragment.

A particular object of this invention resides in a method of selecting biologically active compounds, said method comprising contacting in vitro a test compound with a SPOCK2 gene or SPOCK2 polypeptide, preferably a SPOCK2 gene or a SPOCK2 polypeptide, or a fragment thereof of at least 15 consecutive residues, comprising at least one polymorphism according to the invention according to the present invention, and determining the ability of said test compound to bind said SPOCK2 gene or SPOCK2 polypeptide. Binding to said gene or polypeptide provides an indication as to the ability of the compound to modulate the activity of said target, and thus to affect a pathway leading to bronchopulmonary dysplasia in a subject. In a preferred embodiment, the method comprises contacting in vitro a test compound with a SPOCK2 polypeptide or a fragment thereof, preferably a SPOCK2 polypeptide or a fragment thereof comprising at least one polymorphism according to the invention according to the present invention, and determining the ability of said test compound to bind said SPOCK2 polypeptide or fragment. The fragment preferably comprises a metalloproteases-binding site of the SPOCK2 polypeptide.

A particular object of this invention resides in a method of selecting compounds active for the treatment of bronchopulmonary dysplasia, said method comprising contacting in vitro a test compound with a SPOCK2 polypeptide or a fragment thereof of at least 15 consecutive residues, preferably a SPOCK2 polypeptide or a fragment thereof comprising at least one polymorphism according to the invention according to the present invention, and determining the ability of said test compound to bind said SPOCK2 polypeptide or fragment thereof. The SPOCK2 polypeptide or fragment thereof may be used in essentially pure form, in suspension, or immobilized on a support.

In a further particular embodiment, the method comprises contacting a recombinant host cell expressing SPOCK2 polypeptide, preferably a SPOCK2 polypeptide comprising at least one polymorphism according to the invention according to the present invention, with a test compound, and determining the ability of said test compound to bind said SPOCK2 polypeptide and/or to modulate the activity of SPOCK2 polypeptide.

The determination of binding may be performed by various techniques, such as by labelling of the test compound, by competition with a labelled reference ligand, two-hybrid Screening Assay, etc. Modulation of activity includes, without limitation, the inhibition or activation of the cholesterol uptake and efflux

A further object of this invention resides in a method of selecting biologically active compounds, said method comprising contacting in vitro a test compound with a SPOCK2 polypeptide, preferably a SPOCK2 polypeptide comprising at least one polymorphism according to the invention according to the present invention, and determining the ability of said test compound to modulate the activity of said SPOCK2 polypeptide.

A further object of this invention resides in a method of selecting biologically active compounds, said method comprising contacting in vitro a test compound with a SPOCK2 gene, preferably a SPOCK2 gene comprising at least one polymorphism according to the invention according to the present invention, and determining the ability of said test compound to modulate the expression of said SPOCK2 gene.

The invention also concerns methods of selecting biologically active compounds using a non-human transgenic animal expressing a SPOCK2 protein, preferably a SPOCK2 protein comprising at least one polymorphism according to the invention according to the present invention. Optionally, said non-human transgenic animals can be homozygote or heterozygote for the altered SPOCK2 gene. Said methods comprise (i) administrating a test compound to said non-human transgenic animal, and (ii) determining the ability of said test compound to modulate the SPOCK2 activity or expression (mRNA or protein). Said SPOCK2 activity can be assessed by evaluating downstream protein (i.e. metalloproteinase) expression or activity.

The above screening assays may be performed in any suitable device, such as plates, tubes, dishes, flasks, etc. Typically, the assay is performed in multi-wells plates. Several test compounds can be assayed in parallel.

Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance. The test compounds can be an antisense or an RNAi.

The invention will be further illustrated by the following figures and examples. However, these examples and figures should not be interpreted in any way as limiting the scope of the present invention.

### FIGURES

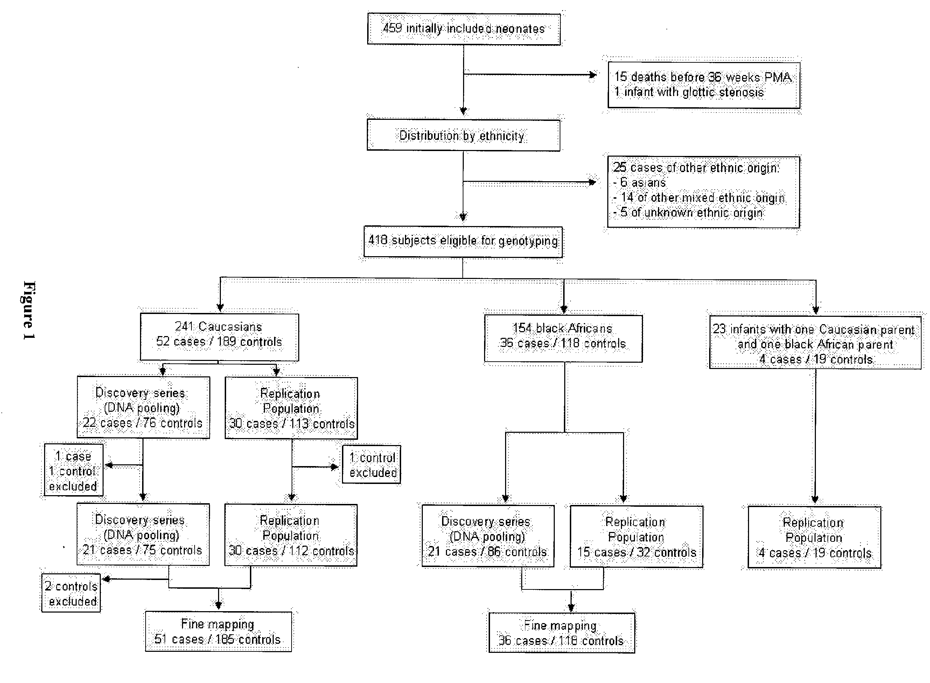

**Figure 1****:** Flowchart of the study population.

### EXAMPLE: IDENTIFICATION OF SPOCK2 AS A SUSCEPTIBILITY GENE FOR BRONCHOPULMONARY DYSPLASIA: A GENOME-WIDE ASSOCIATION STUDY

### Material & methods:

Study design: The study was approved by the local ethics committee (CPP Île de France IX), and written informed consent was obtained from the parents. DNA samples were prospectively collected from cord blood of premature neonates with a gestational age below 28 weeks. The neonates were divided into two discovery series and an independent replication series. The discovery phase consisted of a GWAS based on a DNA pooling strategy in a series of Caucasian infants and a series of black African infants. The choice to study two ethnic groups of infants was justified by the similar rate of BPD in the two groups, and by the assumption that major genetic BPD susceptibility factors are not influenced by ethnicity. All SNPs convincingly associated with BPD in the two populations were validated by individual genotyping and were replicated in a third independent population. Genes associated with BPD in both pooling groups and in the replication population were then investigated in neonatal rats, focusing on lung developmental gene expression patterns and on changes in gene expression during distal lung development in healthy controls and animals exposed to hyperoxia.

**Study population:** Study population was composed of 418 inborn neonates from 3 neonatal intensive care units. Inclusion and exclusion criteria and a flowchart of the study population are given in the Supplementary Appendix. All the neonates received prophylactic surfactant administration immediately after birth, in the delivery room. The following perinatal factors potentially influencing the risk of BPD were collected: documented maternofetal bacterial infection; antenatal steroid therapy; histologically confirmed chorioamnionitis; the need for a second dose of surfactant; persistent ductus arteriosus requiring medical or surgical treatment; and documented postnatal sepsis. The duration of ventilatory support was not analyzed, because management evolved during the study period. BPD was defined by the continued need for oxygen supplementation at 36 weeks PMA, based on the physiologic test validated by Walsh (Walsh MC, Yao Q, Gettner P, Hale E, Collins M, Hensman A, et al. Impact of a physiologic definition on bronchopulmonary dysplasia rates. Pediatrics. 2004;114(5):1305-11.).

Ethnicity was determined from the ethnic origin of the two parents. The final study population consisted of 241 Caucasian infants, 154 black African infants, and 23 infants with one Caucasian parent and one black African parent.

**DNA pooling:** The first discovery series consisted of 22 Caucasian cases and 76 Caucasian controls, and the second of 21 black African cases and 86 black African controls. Equimolar amounts of each DNA sample were added to the case or control pool in each series. Several independent sets of pools were constructed to control for experimental error. In the Caucasian series, 3 sets of identical pools were constructed, with one in double quantity in order to hybridize it twice, giving a total of 4 independent replicates. In the black African series, 4 sets of identical pools were constructed and each was hybridized once, again yielding 4 independent replicates. Further details on DNA pools construction are given in the Supplementary Appendix.

**DNA pool genotyping:** Genotyping was performed with the Infinium II Illumina HumanHap300 Genotyping BeadChip array for the Caucasian population and the Illumina HumanHap650Y array for the black population. Each replicate was hybridized once, meaning that 4 arrays were used for each case and control pool in each population. A total of 16 arrays were thus used. Raw and transformed data sets for each microarray experiment are available at http://www.ncbi.nlm.nih.gov/geo/query/acc.cgi under the Accession Number GSE22284. Quality controls and the analytical strategy are described in the Statistical Analysis section.

**Fine mapping and individual genotyping of the SPOCK2 region:** The HapMap CEU data identified 70 SNPs with a minimum minor allele frequency (MAF) of 0.05 in a region spanning 20 kb upstream and 20 kb downstream of the SPOCK2 gene. Haploview version 4.1 selected 29 SNPs that captured the 70 alleles at r²=1. Genotyping was performed by Integragen with Golden Gate Illumina technology. Before analyzing the results, several quality controls (QC) were implemented: SNPs showing deviation from Hardy-Weinberg equilibrium in the controls (p < 0.05) or with genotyping success rates of less than 90% were excluded from analysis, as were individual DNA samples for which the genotyping success rate across all SNPs was less than 70%. Twenty-two SNPs passed the QC procedure and three DNA samples were excluded.

**Rat lung tissue collection and lung cell isolation:** All animal experiments complied with the Guide for Care and Use of Laboratory Animals and were authorized by the French Ministry of Agriculture. For mRNA expression profiles, lung tissues were collected between fetal day 20 and postnatal day 28, and also from adult rats (8 weeks of age). For immunofluorescence analysis, lungs were extracted at postnatal days 7 and 14. Airway epithelial cells (AECs) and fibroblasts were isolated by differential adhesion and centrifugation as described elsewhere (Chelly N, Mouhieddine-Gueddiche OB, Barlier-Mur AM, Chailley-Heu B, Bourbon JR. Keratinocyte growth factor enhances maturation of fetal rat lung type II cells. Am J Respir Cell Mol Biol. 1999;20(3):423-32.), on postnatal days 1, 7, 14 and 21. It has been shown that cells isolated by this procedure display morphologic features characteristic of their respective cell types (Boucherat O, Bourbon JR, Barlier-Mur AM, Chailley-Heu B, D'Ortho MP, Delacourt C. Differential expression of matrix metalloproteinases and inhibitors in developing rat lung mesenchymal and epithelial cells. Pediatr Res. 2007;62(1):20-5.).

**Hyperoxic exposure of rat pups:** Exposure to hyperoxia induces alveolar growth disorders in newborn rats (Boucherat O, Franco-Montoya ML, Thibault C, Incitti R, Chailley-Heu B, Delacourt C, et al. Gene expression profiling in lung fibroblasts reveals new players in alveolarization. Physiol Genomics. 2007;32(1):128-41). Rat pups and their dams were exposed in parallel to >95% FiO₂ or room air from day 5 to day 10. On day 10, the pups were killed; lungs were extracted, then immediately frozen in liquid nitrogen and stored at -80°C until RNA extraction. Further details on animal experiments are given in the Supplementary Appendix.

**Real-time quantitative PCR (qPCR):** Real-time qPCR was performed on an ABI Prism 7000 (Applied Biosystems). Primers were designed to be intron-spanning to avoid co-amplification of genomic DNA, using Primer Express software (Applied Biosystems) (see supplementary appendix for further information).

**Immunofluorescence analysis:** Co-immunofluorescence experiments were performed using a monoclonal mouse anti-human SPOCK2 antibody and a polyclonal rabbit anti-bovine SPB antibody or a polyclonal rabbit anti-human collagen IV antibody on rat lung tissue frozen sections (see supplementary appendix for further information).

### Statistical analysis:

### A. DNA pooling data

Data obtained with the two series of pools were analyzed as follows: SNPs with MAF among the controls <0.05 or >0.95 were excluded from the analysis, as were the 5% of SNPs showing the highest variability in allele frequency in controls.

Selection of SNPs for individual genotyping was based on two different methods.

### 1) Allele frequency difference method

This method was based on the rank of the SNPs according to the allelic differences between cases and controls, to select the top-ranked 1%. In order to select SNPs that yielded the highest allelic differences while being highly replicable, we arbitrarily paired each case replicate with a control replicate, computed the differences in MAF for each pair (4 in each population), and ranked the differences (4 ranks for each population). For each series, we selected the SNPs that were among the best 1% in all four rankings.

### 2) Combined Z-score analysis

For each SNP, we computed a Z-score and a p value as described by Abraham et al. [17], combining experimental and sampling errors. We selected SNPs with an uncorrected p value of <10⁻⁶ for each series.

Because different chips were used for the two discovery series, and because a given locus may be identified by different SNPs in ethnically different populations, we used a locus-specific selection method. We considered that the identification of at least two significant SNPs within 30 kb [26] in both discovery populations reflected a single locus. To be selected for replication step, a gene should have a locus identified by both statistical methods in both sets of pools representing two different populations.

### B. Individual genotyping

For each SNP, two types of association with BPD were tested: the minor allele frequency by analysis of variance, and the genotype distribution by using the chi-squared test or Fisher's exact test. Associations between BPD and perinatal factors were identified by using the chi-squared test for qualitative variables and analysis of variance for quantitative variables. Odds ratios (OR) for SNPs with p <0.05 in at least one of the two tests were then computed with a logistic regression model that included clinical factors with p values below 0.1 in univariate analysis. After logistic regression analyses, p values below 0.05 were considered to indicate statistical significance. Linkage disequilibrium analysis was implemented with Haploview software version 4.1.

### C. Animal studies

Differences between treatment groups were evaluated with the Kruskal-Wallis non-parametric test for multiple group comparisons and the Mann-Whitney U test for two-group comparisons. P values below 0.05 were considered to indicate statistical significance.

### Supplementary appendix :

### Study population

### Inclusion criteria:

Gestational age below 28 weeks PMA

### Inborn birth

Endotracheal intubation in the delivery room with prophylactic instillation of surfactant
Written inform consent obtained from the parents

### Exclusion criteria:

Postnatal transfer to one of the 3 participating centers
Lethal malformation
Pulmonary malformation
Hemodynamic failure resistant to treatment
Absence of parental consent

The flowchart of the study population is shown in Figure 1.

**DNA pools contruction:** Genomic DNA was extracted from cord-blood leukocytes with the Nucleon kit (Amersham). Pool construction and hybridization were performed in collaboration with Integragen (Genopole, Evry, France). The DNA concentration was determined by UV spectroscopy at 260 nm before adjustment to 25 ng/µl. DNA quality was assessed by electrophoretic migration on 1% agarose gel. Four replicate dilutions were prepared from each sample. The concentration of each dilution of the first 24 samples was determined twice by fluorescence dosage with Sybr Green I and twice by RNAse P qPCR. The two different methods gave similar results, and dosage with Sybr Green I was chosen for the rest of the samples. The coefficient of variation of the 8 Sybr Green I assays for each sample was always less than 3%. After these quality control steps, 2 samples (from a Caucasian case and a Caucasian control) were excluded because of insufficient DNA. The accuracy and repeatability of pipetting was checked by a fluorescent test on the 10-µl pipette used for pool construction

**Rat lung tissue collection and lung cell isolation:** Dated pregnant Sprague-Dawley rats were purchased from Charles River (Saint Germain sur l'Arbresle, France). For all animal experiments, day 0 of gestation was the day of mating, and normal term was 22 days. Pups and adult rats were killed by intraperitoneal sodium pentobarbital injection (70 mg/kg, Ceva, Libourne, France) and were exsanguinated by aortic transection. Fetuses were retrieved by Cesarean section under pentobarbital anesthesia. For mRNA expression profiles, lungs were extracted, immediately frozen in liquid nitrogen and kept at -80°C until RNA extraction. For immunofluorescence analysis, lungs were perfused with Tissue-Tek O.C.T. diluted in PBS via a polyethylene tracheal cannula, then frozen in liquid nitrogen and stored at -80°C until tissue section.

**Hyperoxic exposure of rat pups:** Rat pups and their dams were placed in Plexiglas exposure chambers (Charles River) and run in parallel with either > 95% or 21% (room air) FiO₂ from day 5 to day 10. The oxygen concentration was regularly monitored. Because adult rats have limited resistance to high O₂, the dams were switched daily between O₂-exposed and room air-exposed litters. The chambers were opened for 20 min every day to switch dams between the air and O₂ environments and to clean the cages. On day 10, the pups were killed and the lungs were extracted as described above. Tissues were then immediately placed in liquid nitrogen and kept at -80°C until RNA extraction.

**RNA Isolation and Reverse Transcription:** Total RNA was extracted from fetal sheep lung tissue using Trizol reagent (Invitrogen, http://www.invitrogen.com) according to the manufacturer's instructions. The pelleted RNA was dissolved in sterile water and quantified by absorbance at 260 nm (BioPhotometer, Eppendorf, http://www.eppendorf.de). RNA quality and integrity were confirmed after electrophoresis of 11g of each sample in 1% agarose gel. RNAs were reverse-transcribed into cDNAs using 2 lg of total RNA, Superscript II reverse transcriptase, and random hexamer primers (Invitrogen), according to the manufacturer's protocol.

**Real-time quantitative PCR (qPCR):** Real-time PCR was performed using the following protocol: initial denaturation (10 min at 95°C), then two-step amplification (15 sec at 95°C followed by 1 min at 60°C) repeated 40 times. Melting curve analysis was used to check that a single specific amplicon was generated. Reaction mixtures consisted of 25 ng of cDNA, SYBR Green 2X PCR Master Mix (Applied Biosystems), and forward and reverse primers in a volume of 25 µl. Real-time quantification was achieved by measuring the increase in fluorescence caused by SYBR Green dye binding to double-stranded DNA at the end of each amplification cycle. Relative expression was determined by using the ΔΔCt (threshold cycle) method for normalized samples (ΔCt) relative to a calibrator sample, according to the manufacturer's protocol. Each PCR run included a no-template control and a sample without reverse transcriptase. All measurements were performed in triplicate.

**Immunofluorescence analysis:** After fixation for 15 min with 4% paraformaldehyde in PBS, frozen 5-µm tissue sections were permeabilized by incubation in 0.2% Triton in PBS for 10 min. Non-specific binding was blocked by incubation with 3% serum albumin + 10% donkey serum in PBS for 30 minutes at room temperature. Tissue sections were then incubated with a monoclonal mouse anti-human SPOCK2 antibody (1/50, Abnova) and polyclonal rabbit anti-bovine SPB (1/1000, provided by Dr J. A. Whitsett), or a polyclonal rabbit anti-human collagen IV antibody (1/100, Abcam) overnight at 4°C. After washing in PBS, the sections were incubated with Alexa Fluor 488 donkey anti-mouse IgG (green labeling, 1/200, Invitrogen) and Alexa Fluor 555 donkey anti-rabbit IgG (red labelling, 1/200, Invitrogen) at room temperature for 1 hour. Nuclei were labelled with 1/1000 ToPRO3 in PBS for 15 minutes. Control tissue sections were incubated with non-specific mouse and rabbit purified IgGs at the same concentration as the primary antibodies. Images were recorded with a Leica TCS SP2 confocal microscope (Leica Microsystems) equipped with a x63 oil-immersion objective (Numerical Aperture=1.32). The three channels were acquired sequentially with the following excitation and emission parameters: 488 and 500-540 nm for Alexa Fluor 488, 543 and 555-615 nm for Alexa Fluor 555, and 633 and 645-750 nm for ToPRO3. Gains were adjusted to avoid saturation pixel intensities. The three channels were colour-coded (green, red, and blue) and merged.

### Results :

**Study population:** The 418 neonates had a mean gestational age and birth weight of 26.4 ± 0.1 weeks (range 23.6-27.9) and 845 ± 9 g (range 460-1410), respectively. Ninety-one infants (22%) were diagnosed with BPD (22% in Caucasians and 23% in black Africans). In the overall study population, the following 5 clinical factors had p values below 0.1 for the association with BPD and were thus used as adjustment covariates for genetic analyses: birth weight (OR (95% CI) = 0.996 (0.995-0.998), p<0.001), male gender (1.6 (0.99-2.56), p=0.05), the need for a second dose of surfactant (2.6 (1.59-4.22), p<0.001), persistent ductus arteriosus requiring surgical treatment (6.62 (3.19-13.74), p<0.001), and postnatal sepsis (2.4 (1.49-3.98), p<0.001).

**DNA pooling:** The allele frequency difference method selected respectively 583 SNPs in 388 genes and 1458 SNPs in 933 genes in the Caucasian and black African series of pools, 105 genes being common to the two populations. Identical SNPs were identified in 4 genes in the two populations (*IRF2, KIT, SPOCK2* and *LRRC4C*) and 25 genes with SNPs less than 30 kb apart in the 2 populations were selected (Table 1).

**Table 1: Genes and SNPs selected by the allele frequency difference method in the Caucasian and black African pools. This table shows all genes that are common to Caucasian and black African DNA pooling studies with identical SNPs or SNPs distant from less than 30 kb, identified by the allele frequency difference method. Bold rows indicate genes with identical SNPs between the 2 series of pools.**

| | | Caucasian Pool Analysis | | | | Black African Pool Analysis | | | |
|---|---|---|---|---|---|---|---|---|---|
| Gene symbol | Chromosome | db SNP | Position | SNP type | Allelic diff | db SNP | Position | SNP type | Allelic diff |
| ARHGAP 15 | 2 | rs187133 | 143636856 | A/G | 0.339 | rs6756021 | 143644152 | A/C | 0.245 |
| VIT | 2 | rs7560249 | 36851578 | T/C | 0.246 | rs12987048 | 36878217 | A/G | -0.244 |
| **IRF2** | **4** | **rs793801** | **185618577** | **T/C** | **0.223** | **rs793801** | **185618577** | **T/C** | **-0.251** |
| **KIT** | **4** | **rs6820303** | **55236165** | **A/G** | **0.306** | **rs6820303** | **55236165** | **A/G** | **-0.376** |
| | | | | | | **rs12503461** | **55234021** | **T/C** | **-0.305** |
| JAKMIP1 | 4 | rs4689334 | 6136444 | A/G | 0.352 | rs6446452 | 6131718 | T/C | 0.246 |
| MBOAT1 | 6 | rs555017 | 20293030 | A/G | 0.233 | rs943573 | 20306824 | A/G | 0.234 |
| STK31 | 7 | rs2112035 | 23738893 | A/G | -0.216 | rs6461727 | 23717047 | T/C | -0.356 |
| | | | | | | rs2058995 | 23723785 | T/G | 0.337 |
| XKR6 | 8 | rs7460507 | 11006485 | T/C | -0.261 | rs10282848 | 11034077 | A/G | 0.231 |
| SH3GL2 | 9 | rs2208485 | 17589212 | A/G | -0.304 | rs10115064 | 17595449 | T/C | -0.253 |
| PALM2 | 9 | rs4978847 | 111559535 | A/G | -0.226 | rs10115465 | 111550763 | A/G | 0.224 |
| PALM2-AKAP2 | 9 | rs7026192 | 111908142 | A/G | 0.263 | rs10980199 | 111898722 | A/G | 0.321 |
| SMARCA2 | 9 | rs3750417 | 2044409 | | -0.239 | rs2066111 | 2063839 | T/C | -0.271 |
| PCSK5 | 9 | rs1416556 | 77803005 | | -0.322 | rs10781324 | 77829016 | T/C | -0.235 |
| **SPOCK2** | **10** | **rs1245560** | **73507426** | **A/C** | **0.376** | **rs1245560** | **73507426** | **A/C** | **0.263** |
| | | **rs1678627** | **73498754** | **T/C** | **-0.224** | | | | |
| | | **rs1049269** | **73490007** | **A/G** | **0.256** | | | | |
| ASCC1 | 10 | rs11000204 | 73608572 | A/G | 0.222 | rs7088203 | 73616594 | A/G | 0.228 |
| PLCE1 | 10 | rs2689694 | 95927934 | A/G | -0.322 | rs12244826 | 95935975 | T/G | 0.256 |
| KIAA1274 | 10 | rs4747046 | 71943558 | T/C | -0.280 | rs4747042 | 71933696 | A/G | -0.268 |
| TMEM16A | 11 | rs11235420 | 69671222 | A/G | -0.256 | rs7102771 | 69673088 | T/C | -0.263 |
| **LRRC4C** | **11** | **rs7926722** | **40152669** | **T/C** | **-0.217** | **rs7926722** | **40152669** | **T/C** | **0.271** |
| MAML2 | 11 | rs4753196 | 95701116 | A/C | 0.230 | rs494901 | 95688991 | T/C | -0.281 |
| HNT | 11 | rs7108867 | 131239179 | A/G | -0.271 | rs10894472 | 131261275 | T/C | -0.364 |
| | | | | | | rs1036433 | 131261645 | T/C | 0.267 |
| MY016 | 13 | rs1072189 | 108219137 | A/G | 0.232 | rs729550 | 108213244 | T/C | -0.263 |
| KLHL1 | 13 | rs2439610 | 69509378 | T/C | -0.233 | rs1441561 | 69491013 | T/C | -0.249 |
| | | | | | | rs1441563 | 69486432 | A/G | -0.360 |
| | | | | | | rs9542167 | 69531078 | T/C | 0.260 |
| ATP8A2 | 13 | rs1924783 | 24883873 | A/G | -0.227 | rs640894 | 24904474 | A/G | -0.266 |
| TMC7 | 16 | rs7188759 | 18933051 | T/C | 0.311 | rs12599856 | 18929949 | A/G | -0.232 |
| | | rs8043993 | 18956879 | A/G | -0.237 | | | | |
| WWOX | 16 | rs1110559 | 77385366 | A/G | 0.219 | rs7201295 | 77360990 | A/G | 0.257 |
| | | | | | | rs7205435 | 77364835 | T/C | 0.252 |
| CTDP1 | 18 | rs485400 | 75569936 | A/G | -0.257 | rs520874 | 75542430 | A/C | -0.408 |
| JMJD2B | 19 | rs2054519 | 5010360 | T/C | -0.280 | rs16992771 | 4997070 | T/C | -0.282 |
| | | | | | | rs2613766 | 5017995 | T/C | 0.232 |
| | | | | | | rs858414 | 5006196 | T/C | 0.323 |
| DIAPH2 | X | rs1569561 | 96209143 | A/G | -0.339 | rs594294 | 96238841 | A/C | -0.321 |

Combined Z-score analysis selected respectively 64 SNPs in 57 genes and 301 SNPs in 257 genes in the Caucasian and black African series of pools, 8 genes being common to the two populations. Among these 8 genes, only *SPOCK2* exhibited SNPs less than 30 kb apart in the 2 populations (Table 2).

**Table 2: Genes and SNPs selected by the combined Z-score analysis in the white and black pools. This table shows all genes that are common to Caucasian and black African DNA pooling studies, identified by the combined Z-score analysis. Bold row indicates the sole gene with SNPs distant from less than 30 kb between the 2 series of pools. Association with BPD was statistically significant for p value < 10-6.**

| | | Caucasian Pool Analysis | | | | Black African Pool Analysis | | | |
|---|---|---|---|---|---|---|---|---|---|
| Gene symbol | Chromosome | db SNP | Position | SNP type | P value | db SNP | Position | SNP type | P value |
| DPP6 | 7 | rs12668613 | 153576455 | T/C | 8.1x10⁻⁷ | rs9649875 | 154251195 | T/C | 9.4x10⁻⁷ |
| PRUNE2 | 9 | rs1361858 | 78657280 | T/C | 5.3x10⁻⁸ | rs1343405 | 78706850 | T/C | 1.1x10⁻⁷ |
| **SPOCK2** | **10** | **rs1245560** | **73507426** | **A/C** | **1.7x10⁻⁷** | **rs896074** | **73502865** | **T/C** | **9,1x10⁻⁷** |
| | | | | | | **rs1049269** | **73490007** | **A/G** | **4.4x10⁻⁹** |
| HPSE2 | 10 | rs3936941 | 100329731 | T/G | 1.6x10⁻⁷ | rs10509722 | 100268007 | T/C | 6.4x10⁻⁷ |
| AGBL1 | 15 | rs10152333 | 84655363 | T/C | 1.9x10⁻⁷ | rs4887450 | 84756882 | A/C | 2.0x10⁻⁷ |
| | | | | | | rs1347234 | 85356886 | A/C | 1.5x10⁻⁷ |
| | | | | | | rs2701405 | 85340922 | A/C | 2.5x10⁻⁸ |
| | | | | | | rs1010419 | 85337105 | A/G | 7.7x10⁻⁹ |
| DIAPH2 | X | rs1569561 | 96209143 | A/G | 2.5x10⁻⁷ | rs5966792 | 96099869 | T/C | 1.5x10⁻⁷ |
| DMD | X | rs1293821 | 32281166 | A/G | 5.9x10⁻⁸ | rs6628738 | 32740780 | A/C | 3.9x10⁻⁷ |
| | | rs5927969 | 32259356 | T/G | 5.8x10⁻¹³ | rs7887541 | 31981425 | T/C | 6.5x10⁻¹³ |
| IL1RAPL1 | X | rs4829242 | 29541451 | T/C | 8.1X10⁻⁸ | rs6630885 | 29440841 | A/G | 4.6x10⁻⁷ |
| | | | | | | rs6526874 | 29436441 | T/C | 5.7x10⁻⁸ |

*SPOCK2* was the only gene selected with identical SNPs or SNPs less than 30 kb apart in both selection methods and in both populations. Comparison of MAF between each control pool and corresponding HapMap data showed high similarity for the 4 SNPs in the *SPOCK2* region identified by pooling analyses (Table 3). As rs1245560 was the only SNP to show similar MAF in the Caucasian and black African control pools (Table 3), it was chosen for validation by individual genotyping in the 2 discovery series and in an independent replication population composed of 49 cases and 163 controls (30 and 112 Caucasians, 15 and 32 black Africans, and 4 and 19 infants with one Caucasian parent and one black African parent).

**Table 3: Comparison of minor allele frequencies between controls pools and HapMap data for the 4 SNPs of SPOCK2 evidenced by DNA pooling. For black African control pool, allele frequencies were compared to both ASW (African ancestry in Southwest USA) and YRI (Yoruba in Ibadan, Nigeria) HapMap data.**

| db SNP | Position | Minor allele | Caucasian control pool | HapMap CEU data | black African control pool | HapMap ASW/YRI data* |
|---|---|---|---|---|---|---|
| rs1245560 | 73507426 | C | 0.4625 | 0.518 | 0.497 | 0.519/0.576 |
| rs1049269 | 73490007 | G | 0.5809 | 0.598 | 0.8302 | 0.898/0.987 |
| rs1678627 | 73498754 | C | 0.6574 | 0.606 | 0.9344 | 0.811/0.858 |
| rs896074 | 73502865 | C | 0.4580 | 0.403 | 0.1375 | 0.123/0.009 |

**Individual genotyping of rs1245560 in the discovery series:** Individual genotyping of rs1245560 in DNA samples used for pooling studies confirmed the results obtained by pooling analyses. The C allele frequencies were 0.479 and 0.512 in the Caucasian and black African controls, respectively, compared to 0.75 (p=0.003) and 0.737 (p=0.01) in the Caucasian and black African BPD neonates, respectively. Results were also significant when genotype distribution between cases and controls was tested, with p values of 0.01 for rs1245560 in the Caucasian population and 0.004 in the black African population.

**Replication population:** Individual genotyping of rs1245560 also showed a significant association with BPD in the replication series (Table 4).

**Table 4: Individual genotyping of rs1245560 in the replication series. * Individual genotyping of rs1245560 failed for one DNA sample. † OR adjusted for birth weight, gender, patent ductus arteriosus, postnatal sepsis, and additional surfactant**

| | Cases n=49 | Controls n=162* | p value (univariate) | Adjusted OR (95% CI)^{†} | p value |
|---|---|---|---|---|---|
| C allele frequency | 0.592 | 0.475 | 0.049 | 3.57 (1.19-10.76) | 0.0235 |
| AA genotype, n (%) | 11 (22) | 44 (27) | | | |
| AC genotype, n (%) | 18 (37) | 82 (51) | | 0.71 (0.23-1.98) | NS |
| CC genotype, n (%) | 20 (41) | 36 (22) | 0.0342 | 3.03 (1.06-8.65) | 0.0386 |

**Fine mapping:** To confirm and further explore the association *of SPOCK2* with BPD, we selected and genotyped 28 additional flanking SNPs to finely map the region of interest. Fine mapping was performed separately on all the Caucasian samples (51 cases and 185 controls) and all the black African samples (36 cases and 118 controls). Univariate analysis with a p cut-off of 0.05 identified 16 additional SNPs in Caucasians and one in black Africans. Adjusted ORs were then calculated for each SNP by using a logistic regression model. In the Caucasian population, rs1245560 and 7 other SNPs remained significantly associated with BPD, with ORs ranging from 2.67 to 3.37 for allele frequencies and from 2.16 to 3.15 for the genotype distribution (Table 5).

**Table 5: Associations of the SPOCK2 locus with BPD in the Caucasian population. Each SNP is tested for risk allele frequency differences and genotypes distribution in a regression logistic model including clinical risk factors for BPD with a p value<0.1 in univariate analysis i.e. birth weight, gender, persistent ductus arteriosus, postnatal sepsis, and the need for a second dose of exogenous surfactant.**

| | Caucasian population (51 cases vs 185 controls) | | | | | | | |
|---|---|---|---|---|---|---|---|---|
| SNP | rs1245509 | rs1245576 | rs1049269 | rs1678623 | rs1613186 | rs748035 | rs1245560 0 | rs1245558 |
| Risk allele | C | C | G | T | T | A | C | C |
| Adjusted OR (95% CI) | | 2.98 (1.09-8.16) | 3.18 (1.15-8.76) | 2.88 (1.09-7.63) | 2.67 (1.02-7.02) | 2.89 (1.08-9.73) | 3.37 (1.2-9.4) | |
| p value | NS | 0.0339 | 0.0256 | 0.0335 | 0.0462 | 0.0342 | 0.0207 | NS |
| Genotype 2 2 vs 1 1 | | | | | | | | |
| Adjusted OR (95% CI) | | | | 2.74 (1.05-7.15) | | 2.92 (1.11-7.68) | 2.91 (1.07-7.91) | |
| P | NS | NS | NS | 0.0398 | NS | 0.0304 | 0.0368 | NS |
| Genotype 22 vs [1 2 + 2 2] | | | | | | | | |
| Adjusted OR (95% CI) | 2.22 (1.06-4.67) | 2.75 (1.32-5.74) | 3.15 (1.49-6.67) | | | | 2.89 (1.34-6.21) | 2.16 (1.04-4.49) |
| p value | 0.0356 | 0.0068 | 0.0027 | NS | NS | NS | 0.0067 | 0.0379 |

Both SNPs identified in the African population remained significant (Table 6). When logistic regression analysis was restricted to singletons, the association with BPD was no longer significant for three SNPs (rs1245509, rs1678623 and rs1613186) in Caucasians, while both SNPs identified in the African population remained significant. The 5 SNPs that remained significant in Caucasians were in strong linkage disequilibrium (normalized disequilibrium coefficient values of 0.83 to 1) (Figure 1). As rs1245560 still shared similar allele frequencies among controls in the 2 ethnic groups (0.489 for Caucasians and 0.479 for black Africans), we applied logistic regression analysis to this SNP in the overall study population (91 cases and 322 controls) and obtained ORs (95% CI) of 4.5 (2.13-9.49, p<0.001) and 3.78 (1.83-7.82, p<0.001) for the risk allele frequency and risk genotype, respectively.

**Table 6: Associations of the SPOCK2 locus with BPD in the black African population. Each SNP is tested for risk allele frequency differences and genotypes distribution in a regression logistic model including clinical risk factors for BPD with a p value<0.1 in univariate analysis i.e. birth weight, gender, persistent ductus arteriosus, postnatal sepsis, and the need for a second dose of exogenous surfactant.**

| | African population (36 cases vs 118 controls) | |
|---|---|---|
| SNP | rs1245560 | rs1245540 |
| Risk allele | C | T |
| Adjusted OR (95% CI) | 5.84 (1.61-21.14) | 14.81 (1.42-154.71) |
| p value | 0.0072 | 0.0243 |
| Genotype 2 2 vs 1 1 | | |
| Adjusted OR (95% CI) | 4.55 (1.34-15.42) | |
| p value | 0.0151 | NS |
| Genotype 2 2 vs [1 2 + 2 2] | | |
| Adjusted OR (95% CI) | 4.82 (1.86-12.48) | 3.74 (1.02-13.71) |
| p value | 0.0012 | 0.0462 |

***SPOCK2* expression in rat lung:** Lung SPOCK2 mRNA levels correlated with the postnatal lung growth stage. *SPOCK2* expression increased gradually after birth, reaching levels about tenfold higher on postnatal day 18, remaining high until postnatal day 28, and returning to the neonatal level in adulthood. SPOCK2 mRNA levels were low in isolated fibroblasts until postnatal day 7 and then increased sharply from day 14 to day 21, following a pattern similar to that observed in the whole lung. No major change in SPOCK2 expression was seen in AECs. SPOCK2 protein was strongly expressed throughout the extracellular matrix (ECM), including the basement membrane, as shown by its colocalization with collagen IV. When rat pups were exposed to hyperoxia for 5 days (P5 to P10), SPOCK2 gene expression increased by about 60% relative to untreated controls.

### Discussion:

Recent studies highlighted the contribution of genetic factors to BPD susceptibility. We conducted a genome-wide association study based on DNA pooling in two different ethnic populations. *SPOCK2* was the only BPD-associated gene that emerged in all analyses. Fine mapping of the *SPOCK2* region validated its association with BPD. In addition, *SPOCK2* expression increased during normal rat lung alveolarization and was markedly enhanced by high-level oxygen exposure, which is known to hinder alveologenesis.

Genome-wide association studies have identified a large number of robust associations between specific loci and complex human diseases. However, differences in clinical practice across neonatal intensive care units may hamper such analyses, and environmental stressors must be tightly controlled in order to detect genetic associations in this setting. The relatively low rate of BPD (22%) in our study population reflects good control of these external factors. The prospective design of this multicenter study helped to ensure that the newborns received similar initial management, whether or not they subsequently developed BPD. Furthermore, the need for supplemental oxygen at 36 weeks PMA was determined with the same standardized test in all the participating centres. We only included cases of moderate and severe BPD, based on the National Institute of Child Health and Human Development definition. Indeed, in the twin study recently published by Lavoie et al (Lavoie PM, Pham C, Jang KL. Heritability of bronchopulmonary dysplasia, defined according to the consensus statement of the national institutes of health. Pediatrics. 2008;122(3):479-85.), the relative contributions of genetic and environmental effects seemed to depend on the severity of BPD. Genetic factors appear to play a more important role in moderate and severe BPD than in mild BPD, therefore indicating cases of moderate and severe BPD as a valid outcome to use in genetic association studies.

The DNA pooling study selected one main SNP of interest (rs1245560) in *SPOCK2.* The association of rs1245560 with BPD was confirmed by individual genotyping of all the samples included in the pools, and also in a distinct replication population. Fine mapping of the *SPOCK2* region identified 7 more SNPs significantly associated with BPD. Interestingly, most of these SNPs were in strong linkage disequilibrium. Together with the validation of rs1245560 in the overall study population, these results provided further evidence that *SPOCK2* is a susceptibility locus in BPD.

*SPOCK2* (SPARC/osteonectin, CWCV, and Kazal-like domains proteoglycan 2), also known as Testican-2, is a member of the testican group of extracellular chondroitin/heparan sulfate proteoglycans. *SPOCK2* was originally cloned from a human cDNA library. Although it has been shown in mice that *SPOCK2* is expressed in brain, cerebellum, testis and lung, its role has mainly been explored in the central nervous system. We provide the first strong evidence of a role of *SPOCK2* in normal and abnormal lung development.

Alveolarization takes place between days 4 and 21 in rats and comprises two successive phases: secondary alveolar septation from day 4 to 14, and alveolar wall thinning with fusion of the initially double capillary layer into a single, central microvascular network from day 14 to 21. We found that *SPOCK2* expression was low during the canalicular and saccular stages of rat lung development and started to increase significantly very close to the beginning of alveolarization. Its expression peaked on day 18 and remained elevated until day 28, before falling to the neonatal level in adulthood. This expression profile suggests that *SPOCK2* is involved in the termination of alveolarization rather than its initiation. This is further supported by our results in newborn rats exposed to hyperoxia, as *SPOCK2* expression increased in this model of arrested alveolar development. *SPOCK2* might influence lung development by interacting with matrix metalloproteinases (MMPs). Indeed, both SPOCK1 and SPOCK3 inhibited pro-MMP2 processing by MMP14 and MMP16, and SPOCK2 abolished this inhibition by binding to SPOCK1 or SPOCK 3. We have previously shown the involvement of MMP16 in lung development, with a pattern of expression during lung development very similar to that observed here with SPOCK2. Together with the localization of SPOCK2 in the lung ECM during alveologenesis, these data point to a regulatory role of SPOCK2 in alveolar development.

Fine mapping identified several SNPs associated with BPD, and in close linkage disequilibrium with rs1245560. Among these, rs1049269 seems to be of particular interest. Indeed, it is located in the 3'UTR region and could thus modify micro-RNA (miRNA) binding. MiRNAs are small non-coding RNAs known to regulate gene expression at the post-transcriptional level by inhibiting translation, degradation or storage of mRNA [34]. Several studies have shown the involvement of miRNAs in several monogenic and multifactorial inherited diseases. In particular, SNPs located in or near predicted miRNA target sites within the 3'UTR of mRNAs have the potential to affect the efficiency of miRNA binding to its target site or to create or destroy binding sites. Computational analysis using the Patrocles database (http://www.patrocles.org) revealed that miR-194* binding to the 3'UTR region of SPOCK2 could potentially be modified by rs1049269. Characterization of miRNA expression profiles in human tissues has shown that the group of miR-194 is expressed in normal human lung. Further analysis using the RegRNA database (http://www.RegRNA.org) identified another miRNA, miR-449b, which recognizes a target site immediately upstream of rs1049269. This miRNA is predominantly expressed in trachea, lung and testis.

Thus, a genome-wide association study of a multicenter population of preterm neonates identified SPOCK2 as a new candidate susceptibility locus for BPD. Its pattern of expression during lung development strongly points to a role in alveolarization. In vitro miRNA targeting experiments are underway to identify the potential involvement of rs1049269 in miRNA binding and gene expression level.

### REFERENCES

Throughout this application, various references describe the state of the art to which this invention pertains.

## Claims

1. A method of identifying a premature infant at risk of having or developing bronchopulmonary dysplasia, said method comprising a step consisting of detecting in a sample obtained from said premature infant the single nucleotide polymorphism rs1245560 in the SPOCK2 gene, wherein the presence of allele (C) of rs1245560 indicates an increased risk of having or being at risk of having or developing bronchopulmonary dysplasia.

2. The method according to claim 1 wherein said method further comprises a step consisting of detecting at least one polymorphism in linkage disequilibrium with rs1245560 wherein said polymorphism is selected from the group consisting of rs1245576, rs1049269, rs748035, rs1245558, rs1245540 and wherein ;
- the presence of the allele C of rs1245576
- the presence of the allele G of rs1049269
- the presence of the allele A of rs748035
- the presence of the allele C of rs1245558
- the presence of the allele G of rs1245540
indicate an increased risk of having or being at risk of having or developing bronchopulmonary dysplasia.

3. The method according to claim 1 or 2 wherein said premature infant is healthy at birth, but considered at risk for developing bronchopulmonary dysplasia, based on clinical indicia such as gestational age, intrauterine growth retardation, twin pregnancy, requirement for exogenous surfactant, patent ductus arteriosus, associated sepsis.

## Patentansprüche

1. Verfahren zur Identifizierung eines Frühgeborenen mit dem Risiko einer bestehenden oder sich entwickelnden bronchopulmonalen Dysplasie, welches Verfahren einen Schritt umfasst, der in der Erfassung des Einzelnukleotidpolymorphismus rs1245560 in dem SPOCK2-Gen in einer von dem Frühgeborenen erhaltenen Probe besteht, wobei die Anwesenheit von Allel (C) von rs1245560 ein erhöhtes Risiko einer bestehenden oder ein vorhandenes Risiko einer bestehenden oder sich entwickelnden bronchopulmonalen Dysplasie anzeigt.

2. Verfahren nach Anspruch 1, bei welchem das Verfahren ferner einen Schritt umfasst, der in der Erfassung mindestens eines Polymorphismus in Verbindungsungleichgewicht mit rs1245560 besteht, wobei der Polymorphismus ausgewählt ist aus der Gruppe bestehend aus rs1245576, rs1049269, rs748035, rs1245558, rs1245540, und wobei
- die Anwesenheit von Allel C von rs1245576,
- die Anwesenheit von Allel G von rs1049269,
- die Anwesenheit von Allel A von rs748035,
- die Anwesenheit von Allel C von rs1245558,
- die Anwesenheit von Allel G von rs1245540
ein erhöhtes Risiko einer bestehenden oder ein vorhandenes Risiko einer bestehenden oder sich entwickelnden bronchopulmonalen Dysplasie anzeigen.

3. Verfahren nach Anspruch 1 oder 2, bei welchem das Frühgeborene bei der Geburt gesund ist, jedoch als mit dem Risiko der Entwicklung bronchopulmonaler Dysplasie behaftet betrachtet wird, und zwar basierend auf klinischen Anzeichen, wie etwa Gestationsalter, intrauterine Wachstumsverzögerung, Zwillingsschwangerschaft, Bedarf an exogenem Tensid, offener Ductus arteriosus, verbundene Sepsis.

## Revendications

1. Procédé d'identification d'un enfant prématuré ayant un risque d'avoir ou de développer une dysplasie bronchopulmonaire, ledit procédé comprenant une étape consistant à détecter dans un échantillon obtenu auprès dudit enfant prématuré le polymorphisme mononucléotidique rs1245560 dans le gène SPOCK2, dans lequel la présence d'un allèle (C) de rs1245560 indique un risque accru d'avoir ou ayant un risque d'avoir ou de développer une dysplasie bronchopulmonaire.

2. Procédé selon la revendication 1, dans lequel ledit procédé comprend en outre une étape consistant à détecter au moins un polymorphisme en déséquilibre de liaison avec rs1245560 dans lequel ledit polymorphisme est sélectionné à partir du groupe constitué par rs1245576, rs1049269, rs748035, rs1245558, rs1245540 et dans lequel :
- la présence de l'allèle C de rs1245576
- la présence de l'allèle G de rs1049269
- la présence de l'allèle A de rs748035
- la présence de l'allèle C de rs1245558
- la présence de l'allèle G de rs1245540
indiquent un risque accru d'avoir ou ayant un risque d'avoir ou de développer une dysplasie bronchopulmonaire.

3. Procédé selon la revendication 1 ou 2, dans lequel ledit enfant prématuré est sain à la naissance, mais considéré à risque pour développer une dysplasie bronchopulmonaire, en se basant sur des indicateurs cliniques comme l'âge gestationnel, le retard de croissance intra-utérine, la grossesse gémellaire, l'exigence d'un tensioactif exogène, la persistance du canal artériel, la sepsie associée.
